# EUROPÄISCHE PATENTSCHRIFT

(11) **EP 2 632 959 B1**
(45) Veröffentlichungstag und Bekanntmachung des Hinweises auf die Patenterteilung: **24.04.2019**
(21) Anmeldenummer: 11776384.7
(22) Anmeldetag: 05.10.2011
(51) Int. Cl.: B01J 20/26, C08F 6/00, A61L 15/22, A61L 15/60, C08J 3/12, C08J 3/24

(54) **VERFAHREN ZUR HERSTELLUNG VON VERBESSERTEN ABSORBIERENDEN POLYMEREN MITTELS KRYOGENEM MAHLEN**
METHOD FOR PRODUCING IMPROVED ABSORBING POLYMERS BY MEANS OF CRYOGENIC GRINDING
PROCÉDÉ DE PRODUCTION DE POLYMÈRES ABSORBANTS AMÉLIORÉS PAR BROYAGE CRYOGÉNIQUE

(30) Priorität: 29.10.2010 DE 102010043113
(43) Veröffentlichungstag der Anmeldung: 04.09.2013
(73) Patentinhaber: Evonik Degussa GmbH, 45128 Essen (DE)
(72) Erfinder: LOICK, Christoph, 47918 Tönisvorst (DE); GARTZ, Dominik, 41751 Viersen (DE); WATTEBLED, Laurent, 40589 Düsseldorf (DE); HARREN, Jörg, 52499 Baesweiler (DE)
(74) Vertreter: Evonik Patent Association
(86) Internationale Anmeldenummer: PCT/EP2011/067348
(87) Internationale Veröffentlichungsnummer: WO 2012/055681

(56) Entgegenhaltungen:
- EP-A- 0 876 888
- EP-A1- 1 837 348
- WO-A1-02/100032
- WO-A1-2008/026783

## Beschreibung

Die vorliegende Erfindung betrifft ein Verfahren zur Herstellung von verbesserten absorbierenden Polymeren mittels kryogenem Mahlen vor dem Trocknen, eine Verwendung eines absorbierenden Polymeren, ein Verfahren zur Herstellung eines Verbundes und eine Verwendung eines Verbundes.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Die Herstellung der Superabsorber erfolgt in der Regel durch die radikalische Polymerisation säuregruppen-tragender Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

Die EP 876 888 A1 offenbart ein Verfahren, bei dem das Hydrogel eingefroren wird, und mittels eines aus rotierenden Messern bestehenden Systems zerkleinert und hierauf getrocknet und gemahlen wird. Der Vorteil soll darin bestehen, dass die Porenstruktur erhalten bleibt, da diese eingefroren wird, vor dem ersten Grobzerkleinern. Nachteilig hierbei ist, dass grundsätzlich ein Schäumungsmittel eingesetzt werden muss, um die Oberflächenorenstruktur zu erhalten. Des Weiteren wird ein höherer Anteil an Feinstpartikeln erhalten, die bei diesem Zerkleinern des eingefrorenen Hydrogels anfallen. Ebenfalls ist ein höherer Energieaufwand sowie auch eine komplexere technische Lösung für die Zerkleinerung des gekühlten Hydrogels erforderlich.
WO 02/100032 A1 offenbart ein Verfahren, bei dem zunächst eine Trocknung erfolgt und anschließend eine kryogene Mahlung durchgeführt wird.

WO 2008/026783A1 offenbart wasserabsorbierende Mittel und ein Verfahren zu dessen Herstellung, wobei das Mittel in einem hydrophoben organischen Lösungsmittel durch Umkehrphasensuspensionspolymerisation, dann Trocknen, danach Oberflächenvernetzungsbehandlung und abschließend Versetzen mit einem Mittel zum Erhöhen eines Hohlraum-unter-Druck-Durchschnittradiusindex erhältlich ist.

EP1837348 A1 beschreibt ein Verfahren zur Herstellung von wasserabsorbierenden Superabsorbern, wobei die stationäre Polymerisation in Gegenwart eines wasserunlöslichen, trockenen Pulvers eines wasserabsorbierenden Harzes oder gequollener Gelteilchen eines wasserabsorbierenden Harzes bei einer Temperatur über 100°C durchgeführt wird, mit anschließender Trocknung des Polymers bei 120°C bis 230°C.

Für eine Bildung von sogenannten "absorbierenden" Polymeren ist eine Polymerisation verschiedener Arten von normalerweise wasserlöslichen Monomeren, oft auch zusammen mit wasserunlöslichen Comonomeren in Gegenwart von Vernetzern erforderlich. Die Zugabe der Vernetzer erfolgt während oder nach der Polymerisation. Derartig absorbierende Polymere sind schwachvernetzte, wasserunlösliche Hydrogelpolymere, die im trockenen und im wesentlichen wasserfreien Zustand eine große Fähigkeit zur Wasserabsorption aufweisen. Diese kann ein Mehrfaches ihres Eigengewichts ausmachen. Aufgrund der hohen Absorptionsfähigkeit eignen sich die absorbierenden Polymere zur Einarbeitung in wasserabsorbierende Strukturen und Gegenstände, wie z. B. Babywindeln, Inkontinenzprodukte oder Damenbinden. Diese absorbierenden Polymere werden in der Literatur auch als "Superabsorber" bezeichnet. In diesem Zusammenhang wird auf Modern Superabsorbent Polymer Technology; F.L. Buchholz, A.T. Graham, Wiley-VCH, 1998 verwiesen.

Die Nachfrage nach Hygieneartikeln, die dünner sind, hat in den letzten Jahren deutlich zugenommen. Heute werden überwiegend dünnere Windeln, die weniger Fluff pulp (Flockenzellstoff) enthalten, hergestellt. Die Aufgabe des Zellstoffs muß demgemäß vom absorbierenden Polymer übernommen werden, ohne dass es zu Qualitätseinbußen kommt. Die Herstellung derartiger Polymere erfolgt in Anwesenheit von Lösemitteln, vorzugsweise Wasser.

Um die Eigenschaften, insbesondere die Absorptionseigenschaften, der Superabsorber für einen Einsatz in Hygieneartikeln zu optimieren, werden die Polymerpartikel, nach der Trocknung und nach der Zerkleinerung des erhaltenen Polymergels, modifiziert, vorzugsweise oberflächenmodifiziert. Bei der Oberflächenmodifizierung kann eine Kern-Schale-artige Morphologie entstehen, die insbesondere bei Superabsorberteilchen bevorzugt ist. Diese Modifizierung dient beispielsweise dazu, die Superabsorber mit geruchbindenden Eigenschaften zu versehen, das Staubverhalten der Superabsorber zu verbessern, das Verbacken der Superabsorberpartikel zu vermindern, das Absorptionsvermögen der Superabsorber unter einer Druckbelastung zu verbessern und/oder die Permeabilitätseigenschaften des Superabsorbers vorteilhaft zu beeinflussen.

Allgemein liegt der vorliegenden Erfindung die Aufgabe zu Grunde, ein wasserabsorbierendes Polymer zur Verfügung zu stellen, dass eine verbesserte Quellgeschwindigkeit aufweist unter gleichzeitiger Beibehaltung der Gesamtqualität. Zusätzlich soll eine einfachere technische Lösung für die Zerkleinerung des Hydrogels bereitgestellt werden, die nicht die Nachteile des Standes der Technik aufweist.

Es ist insbesondere eine Aufgabe der vorliegenden Erfindung, ein Verfahren bereitzustellen, nach dem absorbierende Polymere hergestellt werden können, wobei eine besonders hohe Quellgeschwindigkeit gewährleistet werden kann und diese bereits vor der ersten Trocknung erreicht werden kann.

Die Erfindung bietet ein Verfahren zur Herstellung einer superabsorbierenden Polymerzusammensetzung, wobei das Verfahren folgende Schritte umfasst:
(**i**) Mischen von
   (α1) 0,1 bis 99,99 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, , gegebenenfalls teilneutralisiert,
   (α2) 0 bis 70 Gew.-% polymerisierten, ethylenisch ungesättigten, mit (α1) copolymerisierbaren Monomeren,
   (α3) 0,001 bis 10 Gew.-% eines oder mehrerer Vernetzer,
   (α4) 0 bis 30 Gew.-% wasserlöslichen Polymeren, sowie
   (α5) 0 bis 20 Gew.-% eines oder mehrerer Hilfsstoffe, wobei die Summe der Gewichtsmengen (α1) bis (α5) 100 Gew.-% beträgt;
**(ii)** radikalische Polymerisation unter Vernetzung, um ein wasserunlösliches, wässriges unbehandeltes Hydrogel-Polymer zu bilden;
**(iii)** das Grobzerkleinern des unbehandelten Hydrogel-Polymers in Stückchen mit einem Durchmesser im Bereich von 0,1 mm bis 5,0 cm;
(**iv**) Kühlen des vorzerkleinerten unbehandelten Hydrogel-Polymers im Kühlbereich mit mindestens einer Kühlzone über einen Zeitraum von 30sec. bis 3600 sec, wobei der Kühlbereich einen Temperaturgradienten und zusätzlich im Endbereich des Kühlbereichs eine gekühlte Vorrichtung zur Mahlung des zerkleinerten unbehandelten Hydrogel-Polymeren aufweist und als Kühlmittel inerte feste oder flüssige Kältemittel aus der Gruppe von CO, Kohlendioxid, inerte Kohlenwasserstoffe, wie niederkettige Aliphaten (Methan, Ethan, Propan), halogenierte Kohlenwasserstoffe, Stickstoff, Helium, Argon, oder andere inerte bei Temperaturen unter -30°C sich verflüssigenden Gase, oder Mischungen dieser, eingesetzt werden,
   und Mahlen des
   gekühlten unbehandelten Hydrogel-Polymers;
**(v)** das Trocknen des zerkleinerten unbehandelten Hydrogel-Polymers nach dem Mahlen bei einer Temperatur im Bereich von 85 ºC bis 260 ºC und Sieben der getrockneten Stückchen im Bereich von 150 µm bis 850 µm;
**(vi)** Nachvernetzung des Hydrogel-Polymers und
**(vii)** Trocknung und Konfektionierung des wasserabsorbierenden Polymers,
wobei nach dem Schritt (ii) das noch auf Reaktionstemperatur befindliche Hydrogel-Polymer in einem ersten Schritt zerkleinert wird (iii) und daran anschließend in einem Kühlbereich auf -30 °C bis -200 °C gekühlt und weiter zerkleinert wird (iv), wobei das kryogene Mahlen vor dem Trocknen erfolgt.

In einer bevorzugten Ausführungsform umfasst das erfindungsgemäße Verfahren zur Herstellung einer superabsorbierenden Polymerzusammensetzung folgende Schritte:
(**i**) Mischen von
   (α1) 20 bis 98,99 Gew.-%, bevorzugt 30 bis 98,95 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
   (α2) 1 bis 60 Gew.-%, bevorzugt 1 bis 40 Gew.-% polymerisierten, ethylenisch ungesättigten, mit (α1) copolymerisierbaren Monomeren,
   (α3) 0,01 bis 7 Gew.-%, bevorzugt 0,05 bis 5 Gew.-% eines oder mehrerer Vernetzer, (α4) 1 bis 20 Gew.-%, bevorzugt 5 bis 10 Gew.-% wasserlöslichen Polymeren, sowie (α5) 0,01 bis 7 Gew.-%, bevorzugt 0,05 bis 5 Gew.-% eines oder mehrerer Hilfsstoffe, wobei die Summe der Gewichtsmengen (α1) bis (α5) 100 Gew.-% beträgt;
**(ii)** radikalische Polymerisation unter Vernetzung, um ein wasserunlösliches, wässriges unbehandeltes Hydrogel-Polymer zu bilden;
**(iii)** das Grobzerkleinern des unbehandelten Hydrogel-Polymers in Stückchen mit einem Durchmesser im Bereich von 0,1 mm bis 5,0 cm;
**(iv)** Kühlen des vorzerkleinerten unbehandelten Hydrogel-Polymers und Mahlen des gekühlten unbehandelten Hydrogel-Polymers;
**(v)** das Trocknen des zerkleinerten unbehandelten Hydrogel-Polymers nach dem Mahlen bei einer Temperatur im Bereich von 85 ºC bis 260 ºC und Sieben der getrockneten Stückchen auf eine Größe im Bereich von 155 µm bis 850 µm;
**(vi)** Nachvernetzung des Hydrogel-Polymers und
**(vii)** Trocknung und Konfektionierung des wasserabsorbierenden Polymers.

In einer weiteren Ausführungsform wird das gemäß dem erfindungsgemäßen Verfahren hergestellte Produkt mit Standardsuperabsorbern in dem Schritt (ii) und/oder (iii) gemischt und dann gemäß den Folgeschritten hergestellt.

Das Zumischen von Standardsuperabsorbern kann in einer weiteren Ausführungsform in den Schritten (v) oder (vi) oder (vii) erfolgen.

In einer weitern Ausführungsform wird das nach dem erfindungsgemäßen Verfahren hergestellte Produkt mit Vorprodukten anderer Standardsuperabsorber in den Schritten (ii) und/oder (iii) gemischt und gemäß den Folgeschritten weiterverarbeitet.

Im erfindungsgemäßen Verfahren wird nach dem Schritt (ii) das Hydrogel-Polymer in einem ersten Schritt zerkleinert und daran anschließend in einem Kühlbereich auf -30°C bis -200°C gekühlt und weiter zerkleinert wird.

Bevorzugt ist die Zumischung des Standardsuperabsorbers nach den Schritten (v) bis (vii). Bevorzugt ist ebenfalls die Zumischung des Standardsuperabsorbers nach dem Schritt (i).

Vorteilhafterweise kann in diesem Schritt die Partikelgröße vor dem Trocknen des Gels eingestellt werden, im Gegensatz zum bisherigen Stand der Technik, wo dies nicht möglich ist.

Erfindungsgemäß wird das zerkleinerte unbehandelte Hydrogel-Polymer im Kühlbereich über einen Zeitraum von 30sec. bis 3600 sec gekühlt, bevor es weiterverarbeitet wird. Als Kühlmittel werden inerte feste oder flüssige Kältemittel aus der Gruppe von CO, Kohlendioxid, inerte Kohlenwasserstoffe, wie niederkettige Aliphaten (Methan, Ethan, Propan), halogenierte Kohlenwasserstoffe, Stickstoff, Helium, Argon, oder andere inerte bei Temperaturen unter -30°C sich verflüssigenden Gase, oder Mischungen dieser, eingesetzt. Bevorzugt sind festes Kohlendioxid sowie flüssiges Helium und Stickstoff.

Gemäß dem erfindungsgemäßen Verfahren weist der Kühlbereich mindestens eine Kühlzone auf. Der Kühlbereich weist einen Temperaturgradienten auf, wobei die niedrigeren Temperaturen am Anfang oder am Ende des Kühlbereichs eingestellt werden können.

In einer weiteren Ausführungsform besteht der Kühlbereich aus mindestens zwei Kühlzonen. Diese können in einer Ausführungsform einen Temperaturgradienten über die beiden Kühlzonen aufweisen, oder aber die erste oder letzte Kühlzone weist den Temperaturgradienten auf und mindestens eine andere hat eine definierte Temperatur.

Die Kühlzonen können erfindungsgemäß in einer Variante gleich lang sein. In einer weiteren Ausführungsform ist die Kühlzone mit dem Temperaturgradienten kürzer ausgebildet als die Kühlzone ohne Temperaturgradient. Die Kühlzonen können mit unterschiedlichen inerten Kältemitteln belegt werden.

Zusätzlich weist der Kühlbereich im Endbereich eine gekühlte Vorrichtung zur Mahlung des zerkleinerten unbehandelten Hydrogel-Polymeren auf. Hierbei kann es sich um Mühlen, Kneter, Extruder, Brecher, Messer oder Walzen handeln. Bevorzugt werden Mühlen, Kneter oder Extruder.

Vorteilhafterweise haben sowohl das wasserabsorbierende Polymer als auch das zerkleinerte unbehandelte Hydrogel-Polymer einen FSR von mindestens 0,3, bevorzugt 0,4 und besonders bevorzugt von 0,6.

Die monoethylenisch ungesättigten, säuregruppenhaltigen Monomere (α1) können teilweise oder vollständig, bevorzugt teilweise, neutralisiert sein. Vorzugsweise sind die monoethylenisch ungesättigten, säuregruppenhaltigen Monomere zu mindestens 10 Mol-%, besonders bevorzugt zu mindestens 25 bis 50 Mol-% und darüber hinaus bevorzugt zu 50 bis 90 Mol-% neutralisiert. Die Neutralisation der Monomere (α1) kann sowohl vor als auch nach der Polymerisation erfolgen. Hierbei erfolgt die Teilneutralisierung zu mindestens 10 Mol-%, besonders bevorzugt zu mindestens 25 bis 50 Mol-% und darüber hinaus bevorzugt zu 50 bis 90 Mol-%. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet.

Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acryl-phosphonsäurederivate bevorzugt.

Als ethylenisch ungesättigte, einen protonierten Stickstoff enthaltende Monomere (α1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydro-sulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Diemethylaminopropyl(meth)acrylamid-Hydrosulfat oder Dimethylaminoethyl-(meth)acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylam-moniumethyl(meth)acrylat-Methosulfat oder Dimethylethylammoniumethyl(meth)acrylat-Etho-sulfat sowie (Meth)acrylamido-alkyldialkylamine in quarternisierter Form, beispielsweise (Meth)acrylamidopro- pyltrimethylammoniumchlorid, Trimethylammoniumethyl(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)- acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylen-glykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxy-ethylen)bis(meth)acrylamid, N,N'-Hexamethylenbis(meth)acrylamid oder N,N'-Methylenbis(meth)acrylamid, Polyalkoxydi(meth)acrylate, beispielsweise Diethylenglykoldi-(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi-(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acry- lat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)-acrylat, Thiopropylenglykoldi(meth)acrylat, Thiopolyethylenglykoldi(meth)acrylat, Thiopoly-propylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammonium-chlorid und Homo- und Copolymere von Diethyl(meth)allyl- aminomethyl(meth)acrylat-ammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri- (meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri-(meth)allylphosphat, Tetra(meth)allylethylendiamin, Poly(meth)allylester, Tetra(meth)allyloxiethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoliglycidylether, Polyethylenglykoldiglycidylether, Glycerindiglycidylether, Glycerinpolyglycidyether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phthalsäurediglycidylester, Adipinsäurediglycidylester, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des Weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat und 2-Hydroxypropyl-(meth)acrylat sowie hydroxyl- oder aminogruppenhaltige (Meth)acrylamide oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O, NaAl(SO₄)₂ × 12 H₂O, KAl(SO₄)₂ × 12 H₂O oder Al₂(SO₄)₃×14-18 H₂O eingesetzt. Besonders bevorzugt werden Al₂(SO₄)₃ und seine Hydrate als Vernetzer der Vernetzungsklasse IV verwendet.

Die im erfindungsgemäßen Verfahren eingesetzten Superabsorberteilchen sind vorzugsweise durch Vernetzer der folgenden Vernetzerklassen bzw. durch Vernetzer der folgenden Kombinationen von Vernetzerklassen vernetzt: I, II, III, IV, I II, I III, I IV, I II III, I II IV, I III IV, II III IV, II IV oder III IV. Die vorstehenden Kombinationen von Vernetzerklassen stellen jeweils eine bevorzugte Ausführungsform von Vernetzern eines im erfindungsgemäßen Verfahren eingesetzten Superabsorberteilchens dar.

Weitere bevorzugte Ausführungsformen der im erfindungsgemäßen Verfahren eingesetzten Superabsorberteilchen sind Polymere, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Superabsorberteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) sind in den Polymerfeinteilchen vorzugsweise Stellmittel, organische oder anorganische Partikel wie beispielsweise Geruchsbinder, insbesondere Zeolithe oder Cyclodextrine, Hautpflegesubstanzen, oberflächenaktive Mittel oder Antioxidantien enthalten.

Die im erfindungsgemäßen Verfahren eingesetzten Superabsorberteilchen sind vorzugsweise dadurch erhältlich, dass zunächst aus den vorgenannten Monomeren und Vernetzern ein wasserabsorbierendes Polymer (P) in partikulärer Form hergestellt wird. Die Herstellung dieses als Ausgangsmaterial für die Superabsorberteilchen dienenden Polymers (P) erfolgt beispielsweise durch Massepolymerisation, die vorzugsweise in Knetreaktoren wie Extrudern oder durch Bandpolymerisation erfolgt, Lösungspolymerisation, Spraypolymerisation, inverse Emulsionspolymerisation oder inverse Suspensionspolymerisation. Bevorzugt wird die Lösungspolymerisation in Wasser als Lösemittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Hierzu gehören thermische Katalysatoren, Redoxkatalysatoren und Photoinitiatoren, deren Aktivierung durch energiereiche Strahlung erfolgt. Die Polymerisationsinitiatoren können dabei in einer Lösung erfindungsgemäßer Monomere gelöst oder dispergiert enthalten sein. Bevorzugt ist der Einsatz wasserlöslicher Katalysatoren.

Als thermische Initiatoren kommen sämtliche dem Fachmann bekannte, unter Temperatureinwirkung in Radikale zerfallende Verbindungen in Betracht. Besonders bevorzugt sind dabei thermische Polymerisationsinitiatoren mit einer Halbwertszeit von weniger als 10 Sekunden, darüber hinaus bevorzugt von weniger als 5 Sekunden bei weniger als 180 ºC, darüber hinaus bevorzugt bei weniger als 140ºC. Dabei sind Peroxide, Hydroperoxide, Wasserstoffperoxid, Persulfate sowie Azoverbindungen besonders bevorzugte thermische Polymerisationsinitiatoren. In manchen Fällen ist es vorteilhaft, Mischungen verschiedener thermischer Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, Benzoylperoxid, Lauroylperoxid, Acetylperoxid, Capyrlperoxid, Isopropylperoxydicarbonat, 2-Ethylhexylperoxydicarbonat, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als thermische Polymerisationsinitiatoren bevorzugt: Azo-Verbindungen, wie Azobisisobutyronitrol, Azobisdimethylvaleronitril, 2,2'-Azobis-(2-amidinopropan)dihydrochlorid, Azo-bis-amidinopropan-dihydrochlord, 2,2'-Azobis-(N,N-dimethylen)isobutyramidin-dihydrochlorid, 2- (Carbamoylazo)- isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxkatalysatoren enthalten als oxidische Komponente mindestens eine der oben angegebenen Perverbindungen und als reduzierende Komponente vorzugsweise Ascorbinsäue, Glukose, Sorbose, Manose, Ammonium- oder Alkalimetall-hydrogensulfit, -sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxkatalysators Ascorbinsäure oder Natriumpyrosulfit verwendet. Bezogen auf die bei der Polymerisation eingesetzte Menge an Monomeren wird 1×10⁻⁵ bis 1 Mol-% der reduzierenden Komponente des Redoxkatalysators und 1×10⁻⁵ bis 5 Mol-% der oxidierenden Komponente des Redoxkatalysators eingesetzt. Anstelle der oxidierenden Komponente des Redoxkatalysators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α -Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)malein- imid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclo-hexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.
Bevorzugt wird erfindungsgemäß ein Redoxsystem bestehend aus Wassersoffperoxid, Natriumperoxodisulfat und Ascorbinsäure eingesetzt. In der Regel wird die Polymerisation mit den Initiatoren in einem Temperaturbereich von 0°C bis 90°C initiiert.

Die Polymerisationsreaktion kann durch einen Initiator oder durch mehrere, zusammenwirkende Initiatoren ausgelöst werden. Weiterhin kann die Polymerisation derart durchgeführt werden, dass man zunächst ein oder mehrere Redoxinitiatoren zusetzt. Im weiteren Polymerisationsverlauf werden dann zusätzlich thermische Initiatoren oder Photoinitiatoren appliziert, wobei im Falle von Photoninitiatoren die Polymerisationsreaktion dann durch die Einwirkung energiereicher Strahlung initiiert wird. Auch die umgekehrte Reihenfolge, also die anfängliche Initiierung der Reaktion mittels energiereicher Strahlung und Photoinitiatoren oder thermischen Initiatoren und eine im weiteren Polymerisationsverlauf erfolgende Initiierung der Polymerisation mittels eines oder mehrerer Redoxinitiatoren ist denkbar.

Um die so erhaltenen Polymere (P) in eine partikuläre Form zu überführen, können diese nach ihrer Abtrennung aus der Reaktionsmischung zunächst bei einer Temperatur in einem Bereich von 20 bis 300ºC, bevorzugt in einem Bereich von 50 bis 250ºC und besonders bevorzugt in einem Bereich von 100 bis 200ºC bis hin zu einem Wassergehalt von weniger als 40 Gew.-%, bevorzugt von weniger als 20 Gew.-% und darüber hinaus bevorzugt von weniger als 10 Gew.-%, jeweils bezogen auf das Gesamtgewicht des Polymers (P), getrocknet werden. Die Trocknung erfolgt vorzugsweise in dem Fachmann bekannten Öfen oder Trocknern, beispielsweise in Bandtrocknern, Hordentrocknern, Drehrohröfen, Wirbelbetttrocknern, Tellertrocknern, Paddeltrocknern oder Infrarottrocknern.

Gemäß der vorliegenden Erfindung erfolgt das Zerkleinern dabei vorzugsweise durch Trockenmahlen, vorzugsweise durch Trockenmahlen in einer Hammermühle, einer Stiftmühle, einer Kugelmühle oder einer Walzenmühle.

In einer bevorzugten Ausführungsform der erfindungsgemäßen Verfahren werden als Superabsorberteilchen Partikel eingesetzt, die einen Innenbereich und einen den Innenbereich begrenzenden Oberflächenbereich aufweisen und wobei der Oberflächenbereich eine andere chemische Zusammensetzung als der Innenbereich aufweist oder sich in einer physikalischen Eigenschaft vom Innenbereich unterscheidet. Physikalische Eigenschaften, in denen sich der Innenbereich vom Oberflächenbereich unterscheidet, sind beispielsweise die Ladungsdichte oder der Vernetzungsgrad.

Diese einen Innenbereich und einen den Innenbereich begrenzenden Oberflächenbereich aufweisenden Superabsorberteilchen sind vorzugsweise dadurch erhältlich, dass oberflächennahe, reaktive Gruppen der Superabsorberteilchen vor oder nach ihrer Abtrennung von den übrigen Partikeln des partikulären Polymers (P) nachvernetzt werden. Diese Nachvernetzung kann thermisch, photochemisch oder chemisch erfolgen.

Als Nachvernetzer bevorzugt sind die im Zusammenhang mit den Vernetzern (α3) genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.
Besonders bevorzugt wird Ethylencarbonat als Nachvernetzer eingesetzt.

Bevorzugte Ausführungsformen der Superabsorberteilchen sind diejenigen, die durch Vernetzer der folgenden Vernetzerklassen bzw. durch Vernetzer der folgenden Kombinationen von Vernetzerklassen nachvernetzt sind: II, IV und II IV.

Vorzugsweise wird der Nachvernetzer in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der superabsorbierenden Polymere bei der Nachvernetzung eingesetzt.

Es ist ebenfalls bevorzugt, dass die Nachvernetzung dadurch erfolgt, dass ein Fluid F₁ umfassend ein Lösemittel, vorzugsweise Wasser, mit Wasser mischbare organische Lösemittel wie etwa Methanol oder Ethanol oder Mischungen aus mindestens zwei davon, sowie den Nachvernetzer mit dem Außenbereich der Polymerteilchen bei einer Temperatur in einem Bereich von 30 bis 300°C, besonders bevorzugt in einem Bereich von 100 bis 200°C in Kontakt gebracht werden. Das Inkontaktbringen erfolgt dabei vorzugsweise durch Aufsprühen des Fluids F₁ auf die Polymerteilchen und anschließendes Mischen der mit dem Fluid F₁ in Kontakt gebrachten Polymerteilchen. Dabei ist der Nachvernetzer in dem Fluid F₁ vorzugsweise in einer Menge in einem Bereich von 0,01 bis 20 Gew.-%, besonders bevorzugt in einer Menge in einem Bereich von 0,1 bis 10 Gew.-%, bezogen auf das Gesamtgewicht des Fluids F₁, enthalten. Es ist weiterhin bevorzugt, dass das Fluid F₁ in einer Menge in einem Bereich von 0,01 bis 50 Gew.-%, besonders bevorzugt in einer Menge in einem Bereich von 0,1 bis 30 Gew.-%, jeweils bezogen auf das Gewicht der Polymerteilchen, mit den Polymerteilchen in Kontakt gebracht wird.

Das in den erfindungsgemäßen Verfahren im Verfahrensschritt (vii) eingesetzte Fluid umfasst vorzugsweise ein Lösemittel sowie das vernetzbare, nicht vernetzte Polymer. Als Lösemittel werden vorzugsweise Wasser oder polare, mit Wasser mischbare Lösemittel wie Aceton, Methanol, Ethanol, 2-Propanol oder Mischungen aus mindestens zwei davon eingesetzt. Dabei kann das nicht vernetzte Polymer in dem Lösemittel gelöst oder dispergiert sein.

In einer bevorzugten Ausführungsform des erfindungsgemäßen Verfahrens beinhaltet das Fluid von 18 bis 70 Gew.-% und besonders bevorzugt von 19 bis 55 Gew.-%, jeweils bezogen auf das Fluid, des vernetzbaren, nicht vernetzten Polymers.

Das vernetzbare, nicht vernetzte Polymer basiert vorzugsweise auf
(β1) 20 bis 100 Gew.-%, bevorzugt 50 bis 98,99 Gew.-% und besonders bevorzugt 90 bis 98,95 Gew.-% polymerisierten, ethylenisch ungesättigten, säuregruppenhaltigen Monomeren oder deren Salzen,
(β2) 0 bis 70 Gew.-%, bevorzugt 1 bis 60 Gew.-% und besonders bevorzugt 1 bis 40 Gew.-% polymerisierten, ethylenisch ungesättigten, mit (α1) copolymerisierbaren Monomeren, sowie
(β3) 0 bis 10 Gew.-%, bevorzugt 0,01 bis 7 Gew.-% und besonders bevorzugt 0,05 bis 5 Gew.-% des Monomers, das mit polymerisierten säuregruppentragenden Monomeren, vorzugsweise mit polymerisierten säuregruppenhaltigen Monomeren im Oberflächenbereich der Superabsorberteilchen oder mit anderen polymerisierten säuregruppenhaltigen Monomeren (M) im vernetzbaren, nichtvernetzten Polymer in einer Kondensationsreaktion, in einer Additionsreaktion oder in einer Ringöffnungsreaktion, vorzugsweise bei einem Energieeintrag, reagieren kann,
wobei die Summe der Komponenten (β1) bis (β3) 100 Gew.-% beträgt.

Als Kondensationsreaktionen kommen vorzugsweise die Bildung von Ester-, Amid-, Imid- oder Urethanbindungen in Betracht, wobei die Bildung von Esterbindung bevorzugt ist. Diese Esterbindungen werden vorzugsweise durch die Reaktion einer OH-Gruppe des vernetzbaren, nicht vernetzten Polymers mit einer Säuregruppe des Superabsorberteilchens oder mit einer Säuregruppe des vernetzbaren, nichtvernetzten Polymers gebildet.

Die säuregruppenhaltigen Monomere (β1) sind vorzugsweise zu mindestens 10 Mol-%, besonders bevorzugt zu mindestens 20 Mol-%, darüber hinaus bevorzugt zu mindestens 40 Mol-% und weiterhin bevorzugt im Bereich von 45 bis 80 Mol-% neutralisiert. Die Neutralisation der Monomere kann vor, während oder erst nach der Herstellung des vernetzbaren, nichtvernetzten Polymers erfolgen. Die Neutralisierung erfolgt dabei vorzugsweise mit denjenigen Basen, die bereits im Zusammenhang mit der Neutralisation der säuregruppentragenden Monomere (α1) genannt wurden. Neben den dort genannten Basen werden zur Neutralisation der nicht vernetzten Polymere vorzugsweise auch Basen eingesetzt, die Ammonium, Calcium oder Magnesium als Kationen enthalten. In diesem Zusammenhang bevorzugte Basen sind Ammoniumcarbonat, Ammoniak, Calciumcarbonat, Calciumhydroxid, Magnesiumhydroxid und Magnesiumcarbonat.

Als Monomere (β1) und (β2) werden vorzugsweise diejenigen Monomere eingesetzt, die auch als bevorzugte Monomere (α1) bzw. (α2) eingesetzt werden.

Grundsätzlich kommen als Monomere (M) bzw (β3) alle dem Fachmann geeignet erscheinenden Monomere, insbesondere die der Vernetzerklasse III, in betracht. Bevorzugte Monomere (β3) sind die Umsetzungsprodukte von gesättigten aliphatischen, cycloalphatischen, aromatischen Alkohlen, Aminen oder Thiolen mit ethylenisch ungesättigten Carbonsäuren, reaktiven Carbonsäurederivaten oder Allylhalogeniden. Als Beispiele seien in diesem Zusammenhang genannt: (Meth)allylalkohol, (Meth)allylamin, hydroxyl- oder aminogruppenhaltige Ester der (Meth)acrylsäure, wie Hydroxyalkylacrylate, insbesondere Hydroxymethyl(meth)acrylat, 2-Hydroxyethyl(meth)acrylat oder 2-Hydroxypropyl (meth)acrylat, Aminomalkyl(meth)acrylate, insbesondere Aminomethyl(meth)acrylat, 2-Aminoethyl(meth)acrylat oder 2-Aminopropyl(meth)acrylat, Mono(meth)allylverbindungen von Polyolen, vorzugsweise von Diolen wie beispielsweise Polyethylenglykole oder Polypropylenglykole, sowie Glycidylalkyl(meth)acrylate wie Glycidyl(meth)acrylat.

Besonders bevorzugte vernetzbare, nicht vernetzte Polymere, die in den erfindungsgemäßen Verfahren eingesetzt werden, sind diejenigen Polymere, die auf 1 bis 80 Gew.-%, besonders bevorzugt auf 1 bis 60 Gew.-% und darüber hinaus bevorzugt auf 1 bis 20 Gew.-% (Meth)acrylamid und 20 bis 99 Gew.-%, besonders bevorzugt auf 40 bis 99 Gew.-% und darüber hinaus bevorzugt auf 80 bis 99 Gew.-%, jeweils bezogen auf das Gesamtgewicht des nicht vernetzten Polymers, auf (Meth)acrylsäure basieren, wobei die (Meth)acrylsäure vorzugsweise teilneutralisiert ist.

Es ist weiterhin bevorzugt, dass das in dem erfindungsgemäßen Verfahren eingesetzte Fluid neben dem Lösemittel und dem vernetzbaren, nicht vernetzten Polymer einen weiteren, externen Vernetzer umfasst. Dies gilt insbesondere dann, wenn die vernetzbaren, nicht vernetzten Polymere keine Monomere (M) bzw. (β3) beinhalten. Als weitere externe Vernetzer sind dabei diejenigen der Vernetzerklassen II und IV bevorzugt, die bereits im Zusammenhang mit den Vernetzern (α3) genannt wurden. Besonders bevorzugte weitere Vernetzer sind diejenigen, die als besonders bevorzugte Vernetzer der Klassen II und IV im Zusammenhang mit den Monomeren (α3) genannt wurden. Es ist in diesem Zusammenhang weiterhin bevorzugt, dass das Fluid den weiteren externen Vernetzer in einer Menge in einem Bereich von 0,01 bis 30 Gew.-%, bevorzugt in einem Bereich von 0,1 bis 15 Gew.-% und besonders bevorzugt in einem Bereich von 0,2 bis 7 Gew.-%, bezogen auf das Gewicht des nicht vernetzten Polymers, enthält.

Bevorzugte Additive sind Substanzen, welche die Brüchigkeit der durch das erfindungsgemäße Verfahren hergestellten Superabsorberpartikel reduzieren, wie etwa Polyethylenglykol, Polypropylenglykol, gemischte Polyalkoxylate, auf Polyolen wie Glycerin, Trimethylolpropan oder Butandiol basierende Polyalkoxylate, Tenside mit einem HLB von mehr als 10 wie Alkylpolyglucoside oder ethoxylierte Zuckerester, beispielsweise Polysorbate unter dem Handeslnamen Tween von ICI. Diese Additive wirken teilweise zugleich auch als weitere Vernetzer, wie zum Beispiel Polyethylenglykol, Polypropylenglykol, Trimethylolpropan oder Butandiol.

Weiterhin als Additive bevorzugt sind Mittel, welche die Härte der durch das erfindungsgemäße Verfahren hergestellten Superabsorberpartikel reduzieren, wie etwa kationische Tenside wie Alkyltrimethylammoniumchlorid, Dialkyldimethylammoniumchlorid, Dimethylstearylammoniumchlorid, Alkylbenzyldimethylammoniumchlorid oder die entsprechenden Methylsulfate, quaternäre Tallölfettsäure-Imidazoliniummethosulfate. Diese Additive werden vorzugsweise in Mengen in einem Bereich von 0 bis 5 Gew. %, besonders bevorzugt in einem Bereich von 0,5 bis 4 Gew.-%, bezogen auf das Gewicht des nicht vernetzten Polymers, eingesetzt. Die Additive können dabei entweder vor oder nach der Polymerisation zugesetzt werden. Sie binden die Polycarboxylate durch Anionen-Kationen-Wechselwirkung und bewirken somit den Erweichungseffekt. Sie bewirken gleichzeitig eine Verbesserung der Absorptionsfähigkeit für wässrige Flüssigkeiten. Ein anderer Vorteil der Substanzen ist ihre biozide Wirkung, die einen ungewollten Abbau der Quellungsmittel verhindern. Diese Eigenschaft ist für manche Anwendungen besonders wichtig.

Als Additive sind des weiteren Trennmittel bevorzugt, wie etwa anorganische oder organische pulverförmige Trennmittel. Diese Trennmittel werden vorzugsweise in Mengen in einem Bereich von 0 bis 2 Gew.-%, besonders bevorzugt in einem Bereich von 0,1 bis 1,5 Gew.-%, bezogen auf das Gewicht des vernetzten Polymers, eingesetzt. Bevorzugte Trennmittel sind Holzmehl, Pulp Fasern, pulverförmige Rinde, Cellulosepulver, mineralische Füllstoffe wie Perlit, synthetische Füllstoffe wie Nylonpulver, Rayonpulver, Diatomerde, Bentonit, Kaolin, Zeolithe, Talk, Lehm, Asche, Kohlenstaub, Magnesiumsilikate, Dünger oder Mischungen der Substanzen. Hochdisperse pyrogene Kieselsäure, wie sie unter dem Handelsnamen Aerosil von Evonik Degussa vertrieben wird, ist bevorzugt.

In einer bevorzugten Ausführungsform des erfindungsgemäßen Verfahrens erfolgt das Inkontaktbringen der Superabsorberteilchen mit dem Fluid enthaltend das nicht vernetzte Polymer in Gegenwart eines auf einem Polyzucker oder einer Silizium-Sauerstoff beinhaltenden Verbindung oder einer Mischung von mindestens zwei davon basierenden Effektstoffes. Dabei kann der Effektstoff im Fluid enthalten sein oder aber vor dem Inkontaktbringen der Superabsorberteilchen mit dem Fluid mit den Superabsorberteilchen vermischt werden. Es ist auch möglich, dass der Effektstoff in einem weiteren Fluid F' gelöst oder dispergiert wird und in Form dieser Lösung oder Dispersion zusammen mit dem Fluid mit den Superabsorberteilchen in Kontakt gebracht wird. Dabei umfasst das Fluid F' neben dem Effektstoff vorzugsweise eine Flüssigkeit, wobei als Flüssigkeit Wasser sowie organische Lösemittel wie etwa Methanol oder Ethanol, oder aber Mischungen aus mindestens zwei davon, besonders bevorzugt sind, wobei Wasser als Flüssigkeit besonders bevorzugt ist.

Als Polyzucker kommen erfindungsgemäß alle dem Fachmann geläufigen Stärken und deren Derivate sowie Cellulosen und deren Derivate sowie Cyclodextrine in Betracht, wobei als Cyclodextrine vorzugsweise α-Cyclodextrin, β-Cyclodextrin, γ-Cyclodextrin oder Mischungen aus diesen Cyclodextrinen eingesetzt werden.

Als Silizium-Sauerstoff beinhaltende Verbindungen sind Zeolithe bevorzugt. Als Zeolithe können alle dem Fachmann bekannten synthetischen oder natürlichen Zeolithe eingesetzt werden. Bevorzugte natürliche Zeolithe sind Zeolithe aus der Natrolith-Gruppe Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Als Kationen enthalten die in dem erfindungsgemäßen Verfahren eingesetzten Zeolithe vorzugsweise Alkalimetall-Kationen wie Li⁺, Na⁺, K⁺, Rb⁺, Cs⁺ oder Fr⁺ und/oder Erdalkalimetall-Kationen wie Mg²⁺, Ca²⁺, Sr²⁺ oder Ba²⁺.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie beta-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Vorzugsweise werden die Zeolithe als Partikel mit einer mittleren Partikelgröße in einem Bereich von 1 bis 500 µm, besonders bevorzugt in einem Bereich von 2 bis 200 µm und darüber hinaus bevorzugt in einem Bereich von 5 bis 100 µm eingesetzt.

Die Effektstoffe werden in den erfindungsgemäßen Verfahren vorzugsweise in einer Menge in einem Bereich von 0,1 bis 50 Gew.-%, besonders bevorzugt in einem Bereich von 1 bis 40 Gew.-% und darüber hinaus bevorzugt in einer Menge in einem Bereich von 5 bis 30 Gew.-%, jeweils bezogen auf das Gewicht der Superabsorberteilchen, eingesetzt.

Als keimhemmende Mittel sind grundsätzlich alle gegen grampositive Bakterien wirksamen Stoffe bevorzugt, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'- Methylen-bis(6-brom-4-chlorphenol), 3-Methyl-4-(1-methylethyl)phenol, 2-Benzyl-4-chlorphenol, 3-(4- Chlorphenoxy)-1,2-propandiol, 3-lod-2-propinylbutylcarbamat, Chlorhexidin, 3,4,4'-Trichlorcarbonilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen TM CAT, Cognis GmbH, Düsseldorf/Deutschland). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat. Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2.

Aluminiumhydroxyallantoinat, Aluminiumchloridtartrat, Aluminium- Zirkonium-Trichlorohydrat, Aluminium-Zirkonium-Tetrachlorohydrat, Aluminium-Zirkonium-Pentachlorohydrat und deren Komplexverbindungen z. B. mit Aminosäuren wie Glycin.

Zum Mischen bzw. Besprühen sind alle Vorrichtungen geeignet, die eine homogene Verteilung des Fluids auf oder mit den Superabsorberteilchen erlauben. Beispiele sind Lödige Mischer (hergestellt durch die Firma *Gebrüder Lödige Maschinenbau GmbH*), Gerickc Multi-Flux Mischer (hergestellt durch die Firma *Gericke GmbH*), DRAIS-Mischer (hergestellt durch die Firma *DRAIS GmbH* Spezialmaschinenfabrik Mannheim), Hosokawa Mischer (*Hosokawa Mokron Co., Ltd*.), Ruberg Mischer (hergestellt durch die Firma *Gebr. Ruberg GmbH* & *C0.KG Nieheim*), Hüttlin Coater (hergestellt durch die Firma *BWI Hüttlin GmbH Steinen),* Fließbetttrockner oder Sprühgranulatoren von AMMAG (hergestellt durch die Firma *AMMAG Gunskirchen, Österreich*) oder Heinen (hergestellt durch die Firma *A. Heinen AG Anlagenbau Varel*), Patterson-Kelly-Mischer, NARA-Schaufelmischer, Schneckenmischer, Tellermischer, Wirbelschichttrockner, Schugi-Mischer oder PROCESSALL.

Für das Inkontaktbringen in einer Wirbelschicht können alle dem Fachmann bekannten und geeignet erscheinenden Wirbelschichtverfahren angewandt werden. Beispielsweise kann ein Wirbelschichtcoater eingesetzt werden.

Einen weiteren Beitrag zur Lösung der eingangs beschriebenen Aufgaben liefert ein Verbund, beinhaltend die erfindungsgemäßen Superabsorber bzw. die durch das erfindungsgemäße Verfahren erhältlichen Superabsorber und ein Substrat. Es ist dabei bevorzugt, dass die erfindungsgemäßen Superabsorber und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher den erfindungsgemäßen Superabsorber in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist.
Einen weiteren Beitrag zur Lösung mindestens einer der eingangs genannten Aufgaben liefert ein Verfahren zur Herstellung eines Verbundes, wobei die erfindungsgemäßen wasserabsorbierenden Polymere bzw. die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung mindestens einer der eingangs genannten Aufgaben liefert auch ein Verbund, erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung mindestens einer der eingangs genannten Aufgaben liefern chemische Produkte beinhaltend die erfindungsgemäßen Superabsorber oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Superabsorber oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung mindestens einer der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierenden Mittel oder Pflanzenschutzwirkstoffe über einen durch den Träger kontrollierten Zeitraum abgegeben werden können.

### TESTMETHODEN

Sofern nicht nachfolgend anders angegeben, werden die hierin erfolgten Messungen nach ERT-Verfahren durchgeführt. "ERT" steht für *EDANA Recommended Test und* "EDANA" für *European Disposable and Nonwoven Association.*

### Absorption gegen einen Druck von 0,3 bzw. 0,7 psi (AAP)

Die Absorption unter Druck wurde als AAP (Absorption at Pressure) nach ERT 442-2-02 an der gesamten Kornfraktion bestimmt. Das Flüssigkeitsaufnahmevermögen gegen einen äußeren Druck (Absorption Against Pressure, AAP) wurde gemäß der EDANA-Methode Nr. 442.1-99 bestimmt. 0,90 g der Prüfsubstanz (abgesiebt zwischen 150 und 850µm) wurden in einen Testzylinder mit 60,0 mm Innendurchmesser und einem Siebboden (400mesh) eingewogen (Konzentration: 0,032 g/cm²) und gleichmäßig verteilt. Auf die Prüfsubstanz wird ein zylindrisches Gewicht (21 g/cm² = 0,3 psi oder 50 g/cm² = 0,7psi) mit einem Außendurchmesser von 59,2 mm gelegt. In eine Kunststoffschale werden Filterplatten gelegt, die mit einem Filterpapier abgedeckt werden. Die Kunststoffschale wird mit 0,9 %iger NaCI-Lösung gefüllt, bis der Flüssigkeitsspiegel mit der Oberkante der Filterplatten abschließt. Anschließend werden die vorbereiteten Messeinheiten auf die Filterplatten gestellt. Nach einer Quellzeit von 60 Minuten werden die Messeinheiten entnommen und das Gewicht entfernt. Die aufgenommene Flüssigkeitsmenge wird gravimetrisch ermittelt und auf 1 Gramm Prüfsubstanz umgerechnet.

### Bestimmung der "Free Swell Rate" (FSR)

Die Bestimmung der Absorptionsgeschwindigkeit erfolgte über die Messung der sogenannten *"Free Swell Rate* - *FSR"* gemäß dem in der EP-A-0 443 627 auf der Seite 12 beschriebenen Testverfahren.

### Zentrifugenretentionskapazität (CRC Centrifuge Retention Capacitv)

Die Zentrifugenretentionskapazität wird nach der von der EDANA (European Disposables and Nonwovens Association) empfohlenen Testmethode Nr. 441.2-02 "Centrifuge retention capacity" bestimmt.

Spezifische Oberfläche BET-Messung.

Zur Ermittlung der spezifischen Oberfläche wurde ein Gemini VII 2390 p verwendet, mit einer Evakuierungsrate 1000.00 mmHg/min. Als Adsorptionsmittel wurde Stickstoff verwendet. Die Equilibrierungszeit betrug jeweils 5 sec und der Sättigungsdruck: 756.144 mmHg.

### Beispiele

Eine Monomerlösung bestehend aus 300 g Acrylsäure, die zu 70 Mol-% mit Natronlauge neutralisiert wurde (233,14 g 50%ige NaOH), 442,81 g Wasser, 0,622 g Polyethylenglykol-300-diacrylat, 1.043 g Monoallylpolyethylenglykol-450-monoacrylsäureester wird durch Spülen mit Stickstoff vom gelösten Sauerstoff befreit und auf die Starttemperatur von ca. 4°C abgekühlt. Nachdem die Starttemperatur erreicht wurde, wurde eine Initiatorlösung hinzugegeben (0,3 g Natriumperoxodisulfat in 10 g Wasser, 0,07 g 35%ige Wasserstoffperoxidlösung in 10 g Wasser und 0,015 g Ascorbinsäure in 2 g Wasser). Eine exotherme Polymerisationsreaktion findet statt. Die adiabatische Endtemperatur betrug ca. 105 °C. Das entstandene Hydrogel wurde mit einem Labor-Fleischwolf zerkleinert. Anschließend wurde wie unter a) bis c) beschrieben weiter vorgegangen:

### Referenzprobe

Ohne weitere zusätzliche Behandlung wurde die mit einem Fleischwolf grob zerkleinerte Referenzprobe zwei Stunden bei 150°C im Labor-Umlufttrockenschrank getrocknet.
a) Insgesamt wurden 2 kg eines mit einem Fleischwolf grob zerkleinerten Gels auf zwei große Fotoschalen aufgeteilt. Zuvor wurden 5-15 kg Trockeneispellets in einer Retsch Zentrifugalmühle bei Stufe 2 zu einem feinen Pulver vermahlen. Dieses wurde gleichmäßig auf das noch warme Gel gegeben. Nach ca. 5 Minuten wurde das bereits leicht erstarrte Gel gewendet und erneut mit Trockeneis bedeckt. Dieser Vorgang wurde so oft wiederholt, bis das gesamte Gel erstarrt war (2 bis 4 Wiederholungen). Dann wurde das erstarrte Gel grob händisch zerkleinert, so dass die Bruchstücke in die Retsch Zentrifugalmühle passten. Die Mühle wurde zuvor gekühlt (z.B. mit Trockeneis). Das gefrorene Gel wurde noch zusätzlich mit 1-3 kg Trockeneispellets vermischt. Dieses bewirkt eine Kompensation der Wärmeentwicklung während des Mahlprozesses. Gemahlen wurde bei Stufe 1 und Lochkranz 5mm in der Retsch-Zentrifugalmühle. Nach dem Mahlen lagen recht feine aber noch gefrorene Superabsorberpartikel vor, welche gleichmäßig auf Trockennetze verteilt und anschließend zwei Stunden bei 150°C im Umluft-Labortrockenschrank getrocknet wurden.
b) Es wurde 1 kg eines mit einem Fleischwolf grob zerkleinerten Gels gleichmäßig auf Trockennetze verteilt und zwei Stunden bei 150°C im Umluft-Labortrockenschrank getrocknet. Der getrocknete Superabsorber wurde dann mit ca. 2-5 kg pulverisiertem Trockeneis vermengt. Nach ca. 15-25 Minuten wurden die gefrorenen Superabsorberpartikel mit einer zuvor gekühlten Zentrifugalmühle zerkleinert. Um ein Erwärmen der Mühle während des Zerkleinerungsprozesses zu reduzieren, wurde die Mühle zwischendurch mit 250-500 g Trockeneispellets gekühlt. Es wurde mit Stufe 1 und Lochkranz 5mm zerkleinert. Das Gemisch wurde dann auf zwei Metallschalen verteilt und ca. 2 Stunden bei 50°C in den Labor-Umlufttrockenschrank gegeben.
c) Bei dieser Abänderung in der Verfahrensweise wurde nur die Zentrifugalmühle gekühlt, d.h. das mit einem Fleischwolf grob zerkleinerte Gel wird gleichmäßig auf Trockennetze verteilt und zwei Stunden bei 150 °C im Umluft-Labortrockenschrank getrocknet. Der getrocknete Superabsorber wird mit Trockeneispellets vermischt und sofort anschließend in die zuvor gekühlte Zentrifugalmühle gegeben und gemahlen (Stufe 1, Lochkranz 5mm). Anschließend erfolgte eine Nachtrocknung des leicht angequollenen Superabsorbers durch zweistündige Trocknung bei 50 °C im Labor-Umlufttrockenschrank.

Alle Proben weisen eine Korngrößenverteilung im Bereich von 150 bis 850 µm auf, wobei 80% der Korngrößeverteilung im Bereich von 300 bis 600 µm vorhanden sind.

| Verfahren | CRC | FSR |
|---|---|---|
| | (g/g) | (g/g sec) |
| A | 34,3 | 0,51 |
| Vergleichsbeispiel B | 33,4 | 0,19 |
| Vergleichsbeispiel C | 33,5 | 0,25 |
| Referenz | 33,3 | 0,25 |

Eine Erhöhung der Quellgeschwindigkeit (FSR) wurde für Superabsorberteilchen nach Verfahren a) festgestellt. Referenz und Superabsorber Vergleichsbeispiel b) und Vergleichsbeispiel c) weisen keine erhöhten FSR-Werte auf.

### Ergebnisse nach der Nachvernetzung im Labormaßstab:

Alle Proben wiesen eine Korngrößenverteilung im Bereich von 150 bis 850µm auf, wobei 80% der Korngrößeverteilung im Bereich von 300 bis 600µm vorhanden sind. Die Nachvernetzung erfolgte mittels einer Mischung aus Ethylencarbonat, Aluminiumlactat und/oder Aluminiumsulfat in Wasser bei höheren Temperaturen, wie in der Tabelle unten dargestellt.

Die Referenzprobe, sowie die unter a) bis c) beschriebenen Proben wurden nachvernetzt.

| Verfahren | Temp./Zeit | CRC | AAP | FSR |
|---|---|---|---|---|
| | [°C/min] | (g/g) | (g/g) | (g/g sec) |
| Referenz | 180/30 | 27,0 | 24,9 | 0,19 |
| A | 180/30 | 27,3 | 24,6 | 0,57 |
| Vergleichsbeispiel B | 180/30 | 26,4 | 23,1 | 0,25 |
| Vergleichsbeispiel C | 180/30 | 26,9 | 24,5 | 0,22 |
| Referenz | 170/90 | 26,1 | 24,4 | 0,21 |
| A | 170/90 | 26,4 | 23,9 | 0,57 |
| Vergleichsbeispiel B | 170/90 | 27,5 | 24,8 | 0,23 |
| Vergleichsbeispiel C | 170/90 | 27 | 24,6 | 0,26 |

Nur die Proben, die gem. a) hergestellt wurden, weisen einen erhöhten FSR-Wert auf

### Durchführung der kryogenen Mahlung mit flüssigem Stickstoff als Kühlmittel

Das mit einem Fleischwolf grob zerkleinerte Gel wurde zunächst an der Raumluft leicht abgekühlt. Die Zentrifugalmühle wurde im Vorfeld mittels Trockeneis gekühlt. Kleine noch lauwarme Mengen Gel wurden in einem mit flüssigen Stickstoff gefüllten Dewar-Gefäß gegeben. Diese verweilten solange im Dewar-Gefäß, bis die verstärkte Gasbildung im Dewar nachließ. Das Gel zerfiel dabei in kleinere Stücke. Diese Stücke wurde dann mittels eines Siebs aus dem Dewar Gefäß entnommen und mit der gekühlten Zentrifugalmühle gemahlen (Stufe 1, Lochkranz 5mm). Das zerkleinerte, gefrorene Material wurde aus der Mühle entnommen und gleichmäßig auf Trockennetze verteilt. Anschließend erfolgte eine Trocknung im Labor-Umlufttrockenschrank bei 150 °C über 2 h.

### Resultate:

Alle Proben weisen eine Korngrößenverteilung im Bereich von 150 bis 850µm auf, wobei 80% der Korngrößeverteilung im Bereich von 300 bis 600µm vorhanden sind.

| Methode | Temp./Zeit | CRC | AAP | FSR |
|---|---|---|---|---|
| | [min/°C] | (g/g) | (g/g) | (g/g sec) |
| Referenz | 180/30 | 33,7 | - | 0,27 |
| FI. Stickstoff | 180/30 | 30,4 | - | 0,52 |
| Referenz | 180/30 | 28,8 | 25,5 | 0,21 |
| FI. Stickstoff | 170/90 | 27,0 | 24,1 | 0,55 |
| Referenz | 170/90 | 27,8 | 24,3 | 0,20 |
| FI. Stickstoff | 170/90 | 27,2 | 24,6 | 0,56 |

Gegenüber der Referenzprobe handelt es sich bei der kryogen gemahlenen Probe um ein bei der als Kühlmittel flüssiger Stickstoff verwendet wurde und zu einer Erhöhung des FSR erhöht führte.

In einer weiteren Versuchsreihe wurden unterschiedliche Mischungsverhältnisse von kryogen behandeltem, wasserabsorbierendem Material mit nicht behandeltem, wasserabsorbierendem Material durchgeführt. Zusätzlich wurden Versuche mit nachvernetztem Material, entweder erfindungsgemäß kryogen gemahlen oder unbehandelt, durchgeführt. Die Nachvernetzung erfolgte mittels einer Mischung aus Ethylencarbonat, Aluminiumlactat und/oder Aluminiumsulfat in Wasser bei höheren Temperaturen, wie in der Tabelle unten dargestellt.

In der ersten Serie von Versuchen wurden nicht kryogen behandeltes wasserabsorbierendes Material und kryogen behandeltes wasserabsorbierendes Material bezüglich CRC-, AAP-, und FSR-Werten vermessen. Hierbei zeigte sich, dass das erfindungsgemäß behandelte kryogene Produkt höhere CRC- und FSR-Werte aufwies.

Nach einem Nachvernetzungsschritt wie oben beschrieben wurden die Messungen an den Proben wiederholt. Hierbei zeigte sich, dass erst im Nachvernetzungsschritt ein AAP gemessen werden kann. Die CRC- und FSR-Werte waren vermindert gegenüber den nicht nachvernetzten Materialien.

| Probe | Surface | Temp./Zeit | CRC | AAP | FSR |
|---|---|---|---|---|---|
| | crosslinking | °C/min | (g/g) | (g/g) | (g/g sec) |
| Probe unbehandelt | - | - | 33,7 | - | 0,33 |
| Kryogene Probe | - | - | 34,5 | - | 0,60 |
| Probe unbehandelt | + | 170/90 | 29,7 | 26,4 | 0,23 |
| Kryogene Probe | + | 170/90 | 28,5 | 25,1 | 0,45 |

In der zweiten Serie wurden Mischungen aus nicht kryogen behandeltes wasserabsorbierendes Material und kryogen behandeltes wasserabsorbierendes Material bezüglich CRC-, AAP-, und FSR-Werten vermessen.

| Mischungsverhältnis% | Mischungsverhältnis% | Kenndaten | |
|---|---|---|---|
| unbehandelt | kryogen | CRC(g/g) | FSR(g/g sec) |
| 100 | 0 | 37,7 | 0,33 |
| 0 | 100 | 34,5 | 0,60 |
| 50 | 50 | 34,6 | 0,33 |
| 75 | 25 | 33,8 | 0,28 |
| 90 | 10 | 33,9 | 0,28 |

### Bestimmung der BET-Oberfläche von kryogen gemahlenem Superabsorbermaterial

Zur Ermittlung der spezifischen Oberfläche wurde ein Gemini VII 2390 p verwendet, mit einer Evakuierungsrate 1000.00 mmHg/min. Als Adsorptionsmittel wurde Stickstoff verwendet. Die Equilibrierungszeit betrug jeweils 5 sec und der Sättigungsdruck: 756.144 mmHg. Das Probengewicht der kryo-gemahlen Probe betrug 4.3835 g und das Gewicht der nicht erfindungsgemäß verarbeiteten Probe 5.3201 g. Die spezifische Oberfläche des kryogemahlenen Produktes ist vergrößert gegenüber der Referenzprobe, die nicht kryogen vermahlen wurde, wie der unteren Tabelle zu entnehmen ist.

| Probe | FSR [g/g s] | CRC [g/g] | BET surface area [m²/g] |
|---|---|---|---|
| Probe, kryo-gemahlen | 0,45 | 28,5 | 1,49 |
| Probe, nicht kryo gemahlen | 0,23 | 29,7 | 0,85 |

## Patentansprüche

1. Verfahren zur Herstellung eines wasserabsorbierenden Polymers **dadurch gekennzeichnet, dass** das Verfahren folgende Schritte umfasst:
(**i**) Mischen von
(α1) 0,1 bis 99,99 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, gegebenenfalls teilneutralisiert,
(α2) 0 bis 70 Gew.-% polymerisierten, ethylenisch ungesättigten, mit (α1) copolymerisierbaren Monomeren,
(α3) 0,001 bis 10 Gew.-% eines oder mehrerer Vernetzer,
(α4) 0 bis 30 Gew.-% wasserlöslichen Polymeren, sowie
(α5) 0 bis 20 Gew.-% eines oder mehrerer Hilfsstoffe, wobei die Summe der Gewichtsmengen (α1) bis (α5) 100 Gew.-% beträgt;
**(ii)** radikalische Polymerisation unter Vernetzung, um ein wasserunlösliches, wässriges unbehandeltes Hydrogel-Polymer zu bilden;
**(iii)** das Grobzerkleinern des unbehandelten Hydrogel-Polymers in Stückchen mit einem Durchmesser im Bereich von 0,1 mm bis 5,0 cm;
**(iv)** Kühlen des vorzerkleinerten unbehandelten Hydrogel-Polymers im Kühlbereich mit mindestens einer Kühlzone über einen Zeitraum von 30sec. bis 3600 sec, wobei der Kühlbereich einen Temperaturgradienten und zusätzlich im Endbereich des Kühlbereichs eine gekühlte Vorrichtung zur Mahlung des zerkleinerten unbehandelten Hydrogel-Polymeren aufweist und als Kühlmittel inerte feste oder flüssige Kältemittel aus der Gruppe von CO, Kohlendioxid, inerte Kohlenwasserstoffe, wie niederkettige Aliphaten (Methan, Ethan, Propan), halogenierte Kohlenwasserstoffe, Stickstoff, Helium, Argon, oder andere inerte bei Temperaturen unter -30°C sich verflüssigenden Gase, oder Mischungen dieser, eingesetzt werden,
und Mahlen des gekühlten unbehandelten Hydrogel-Polymers;
**(v)** das Trocknen des zerkleinerten unbehandelten Hydrogel-Polymers nach dem Mahlen bei einer Temperatur im Bereich von 85 ºC bis 260 ºC und Sieben der getrockneten Stückchen im Bereich von 150 µm bis 850 µm;
**(vi)** Nachvernetzung des Hydrogel-Polymers und
**(vii)** Trocknung und Konfektionierung des wasserabsorbierenden Polymers,
wobei nach dem Schritt (ii) das noch auf Reaktionstemperatur befindliche Hydrogel-Polymer in einem ersten Schritt zerkleinert wird (iii) und daran anschließend in einem Kühlbereich auf -30 °C bis -200 °C gekühlt und weiter zerkleinert wird (iv), wobei das kryogene Mahlen vor dem Trocknen erfolgt.

2. Verfahren nach einem der vorhergehenden Ansprüche **dadurch gekennzeichnet, dass** der Kühlbereich mindestens zwei Kühlzonen aufweist.

3. Verfahren nach einem der vorhergehenden Ansprüche **dadurch gekennzeichnet, dass** das wasserabsorbierende Polymer und das zerkleinerte kryogen-behandelte Hydrogel-Polymer einen FSR von mindestens 0,3, bevorzugt 0,4 und besonders bevorzugt von 0,6 aufweisen.

4. Verfahren nach einem der vorhergehenden Ansprüche **dadurch gekennzeichnet, dass** das wasserabsorbierende Polymer mit Standardsuperabsorbern in den Schritt (ii) und/oder (iii) oder Schritt (v) bis (vii) gemischt wird.

5. Verfahren nach einem der vorhergehenden Ansprüche **dadurch gekennzeichnet, dass** das wasserabsorbierende Polymer bevorzugt mit Standardsuperabsorbern den Schritten (v) bis (vii) zugemischt wird.

## Claims

1. Process for producing a water-absorbing polymer, **characterized in that** the process comprises the following steps:
(**i**) mixing
(α1) 0.1 to 99.99% by weight of polymerized, ethylenically unsaturated monomers containing acid groups or salts thereof, or polymerized, ethylenically unsaturated monomers including a protonated or quaternized nitrogen, or mixtures thereof, optionally in partly neutralized form,
(α2) 0 to 70% by weight of polymerized, ethylenically unsaturated monomers copolymerizable with (α1),
(α3) 0.001 to 10% by weight of one or more crosslinkers,
(α4) 0 to 30% by weight of water-soluble polymers, and
(α5) 0 to 20% by weight of one or more assistants, where the sum of the weights (α1) to (α5) is 100% by weight;
**(ii)** free-radical polymerization with crosslinking to form a water-insoluble aqueous untreated hydrogel polymer;
**(iii)** coarse comminution of the untreated hydrogel polymer to give pieces having a diameter in the range from 0.1 mm to 5.0 cm;
(**iv**) cooling the precomminuted untreated hydrogel polymer in the cooling region having at least one cooling zone over a period of 30 sec to 3600 sec, wherein the cooling region has a temperature gradient and additionally, in the end region of the cooling region, a cooled apparatus for grinding the comminuted untreated hydrogel polymer and the coolants used are inert solid or liquid refrigerants from the group of CO, carbon dioxide, inert hydrocarbons such as short-chain aliphatics (methane, ethane, propane), halogenated hydrocarbons, nitrogen, helium, argon, or other inert gases which liquefy at temperatures below -30°C, or mixtures thereof, and grinding the cooled untreated hydrogen polymer;
**(v)** drying the comminuted untreated hydrogel polymer after grinding at a temperature in the range from 85°C to 260°C, and sieving the dried pieces in the range from 150 µm to 850 µm;
**(vi)** postcrosslinking the hydrogel polymer and
**(vii)** drying and finishing the water-absorbing polymer,
wherein, after step (ii), the hydrogel polymer still at reaction temperature is comminuted in a first step (iii) and then cooled in a cooling region to -30°C to -200°C and comminuted further (iv), wherein the cryogenic grinding takes place before the drying.

2. Process according to any of the preceding claims, **characterized in that** the cooling region has at least two cooling zones.

3. Process according to any of the preceding claims, **characterized in that** the water-absorbing polymer and the comminuted cryogenically treated hydrogel polymer have an FSR of at least 0.3, preferably 0.4 and more preferably of 0.6.

4. Process according to any of the preceding claims, **characterized in that** the water-absorbing polymer is mixed with standard superabsorbents in step (ii) and/or (iii) or steps (v) to (vii).

5. Process according to any of the preceding claims, **characterized in that** the water-absorbing polymer is preferably added with standard superabsorbents to steps (v) to (vii).

## Revendications

1. Procédé de fabrication d'un polymère absorbant l'eau, **caractérisé en ce que** le procédé comprend les étapes suivantes :
(i) le mélange de
(α1) 0,1 à 99,99 % en poids de monomères polymérisés éthyléniquement insaturés contenant des groupes acides ou leurs sels ou de monomères polymérisés éthyléniquement insaturés contenant un azote protoné ou quaternisé ou leurs mélanges, éventuellement partiellement neutralisés,
(α2) 0 à 70 % en poids de monomères polymérisés, éthyléniquement insaturés, copolymérisables avec (α1), (α3) 0,001 à 10 % en poids d'un ou de plusieurs agents de réticulation,
(α4) 0 à 30 % en poids de polymères solubles dans l'eau, et
(α5) 0 à 20 % en poids d'un ou de plusieurs adjuvants, la somme des quantités en poids d'(α1) à (α5) étant de 100 % en poids ;
(ii) la polymérisation radicalaire avec réticulation, afin de former un polymère hydrogel non traité aqueux insoluble dans l'eau ;
(iii) le broyage grossier du polymère hydrogel non traité en fragments d'un diamètre dans la plage allant de 0,1 mm à 5,0 cm ;
(iv) le refroidissement du polymère hydrogel non traité pré-broyé dans un domaine de refroidissement comprenant au moins une zone de refroidissement pendant une durée de 30 secondes à 3 600 secondes, le domaine de refroidissement comprenant un gradient de température et en outre dans la zone terminale du domaine de refroidissement un dispositif refroidi pour le broyage du polymère hydrogel non traité broyé, et des agents réfrigérants solides ou liquides inertes du groupe constitué par le CO, le dioxyde de carbone, les hydrocarbures inertes, tels que les composés aliphatiques à chaîne courte (méthane, éthane, propane), les hydrocarbures halogénés, l'azote, l'hélium, l'argon ou d'autres gaz inertes qui se liquéfient à des températures inférieures à -30 °C, ou des mélanges de ceux-ci étant utilisés en tant qu'agent de refroidissement,
et le broyage du polymère hydrogel non traité refroidi ;
(v) le séchage du polymère hydrogel non traité broyé après le broyage à une température dans la plage allant de 85 °C à 260 °C et le tamisage des fragments séchés dans la plage allant de 150 µm à 850 µm ;
(vi) la réticulation secondaire du polymère hydrogel, et
(vii) le séchage et le conditionnement du polymère absorbant l'eau,
après l'étape (ii), le polymère hydrogel se trouvant encore à la température de réaction étant broyé lors d'une première étape (iii), puis refroidi dans un domaine de refroidissement à une température de -30 °C à -200 °C, et davantage broyé (iv), le broyage cryogène ayant lieu avant le séchage.

2. Procédé selon l'une quelconque des revendications précédentes, **caractérisé en ce que** le domaine de refroidissement comprend au moins deux zones de refroidissement.

3. Procédé selon l'une quelconque des revendications précédentes, **caractérisé en ce que** le polymère absorbant l'eau et le polymère hydrogel broyé ayant subi un traitement cryogène présentent un FSR d'au moins 0,3, de préférence 0,4 et de manière particulièrement préférée de 0,6.

4. Procédé selon l'une quelconque des revendications précédentes, **caractérisé en ce que** le polymère absorbant l'eau est mélangé avec des superabsorbants standards à l'étape (ii) et/ou (iii) ou aux étapes (v) à (vii).

5. Procédé selon l'une quelconque des revendications précédentes, **caractérisé en ce que** le polymère absorbant l'eau est de préférence mélangé avec des superabsorbants standards aux étapes (v) à (vii).
